Europäisches Patentamt

(19) European Patent Office

Office européen des brevets

(11) Numéro de publication: **0 246 127**
**B1**

(12) **FASCICULE DE BREVET EUROPEEN**

(45) Date de publication du fascicule du brevet: **26.09.90**

(51) Int. Cl.⁵: **A 61 B 17/36**

(21) Numéro de dépôt: **87400887.3**

(22) Date de dépôt: **17.04.87**

(54) Cathéter cardio-vasculaire pour tir au rayon laser.

(30) Priorité: **29.04.86 FR 8606184**

(43) Date de publication de la demande:
**19.11.87 Bulletin 87/47**

(45) Mention de la délivrance du brevet:
**26.09.90 Bulletin 90/39**

(84) Etats contractants désignés:
**AT BE CH DE ES FR GB GR IT LI LU NL SE**

(56) Documents cités:
**EP-A-0 194 841**
**WO-A-85/02101**
**WO-A-85/05263**
**BE-A- 898 753**
**US-A-3 991 764**

(73) Titulaire: **Boussignac, Georges**
**1 avenue de Provence**
**F-92160 Antony (FR)**
(73) Titulaire: **Labrune, Jean-Claude**
**2, avenue de Guyenne**
**F-92160 Antony (FR)**

(72) Inventeur: **Boussignac, Georges**
**1 avenue de Provence**
**F-92160 Antony (FR)**
Inventeur: **Labrune, Jean-Claude**
**2, avenue de Guyenne**
**F-92160 Antony (FR)**

(74) Mandataire: **Bonnetat, Christian et al**
**CABINET BONNETAT 23, Rue de Léningrad**
**F-75008 Paris (FR)**

Courier Press, Leamington Spa, England.

## Description

La présente invention a pour objet un cathéter utilisable notamment pour la destruction ou la réduction des obstacles dans le système circulatoire (cardio-vasculaire) du corps humain.

On sait que, en raison des habitudes alimentaires des pays occidentaux, l'athérosclérose est une maladie répandue. On la soigne, par exemple par voie chirurgicale, notamment par la mise en place de prothèses de pontages contournant la zone obstruée, ou encore par la dilatation de la sténose athéromateuse au moyen de sondes à ballonnets. Toutefois ces interventions ne sont possibles que dans le cas des grosses artères.

On connaît aussi la mise en oeuvre d'un laser pour détruire localement des tissus, mais l'utilisation pratique des appareils connus se révèle extrêmement coûteuse, car la puissance requise entraîne la destruction de la fibre optique de conduction du rayon laser après quelques tirs; dont le nombre dépend de l'énergie apportée et de la nature et du volume des obstacles.

Cependant, pour les petites artères (en dessous du genou), la chirurgie est jusqu'à présent inefficace. Le système cardio-vasculaire humain est en effet un système complexe, contenant des vaisseaux de diamètres fort différents, allant de l'ordre du millimètre à quelques centimètres.

L'objet de la présente invention est un appareil permettant des tirs répétés, jusqu'à destruction complète de la plaque d'athérome, et cela même dans le cas de petits vaisseaux.

A cette fin, selon l'invention, le cathéter pour le tir au rayon laser est caractérisé en ce que ledit orifice de sortie est relié à un conduit de retour pour ledit fluide de refroidissement optiquement neutre et en ce que ledit conduit de retour s'étend également parallèlement à ladite fibre optique.

Ainsi, grâce audit fluide de refroidissement, ladite fibre optique peut assurer la transmission d'une énergie importante pendant des temps relativement longs. De plus, à cause de sa structure simple, le cathéter selon l'invention peut être adapté aux vaisseaux très petits.

On remarquera que, par le brevet belge BE—A—898 753, on connaît déjà un cathéter du type comportant une fibre optique contenue dans une gaine, ainsi qu'un système optique. Toutefois, dans ce cathéter connu, le système optique est agencé à l'extrémité distale de ladite fibre optique et la gaine est ouverte à son extrémité distale en vue de l'injection d'un fluide dans le système circulatoire. Par suite, l'injection de liquide dans la gaine pour éventuellement refroidir la fibre optique ne peut être réalisée que de façon accessoire et épisodique et qu'avec des liquides tolérés par le système circulatoire. Un tel inconvénient limite dont considérablement l'emploi de ce cathéter antérieur, ainsi que l'énergie que peut transmettre sans détérioration ladite fibre optique.

Du document WO 85/05263 est connu un cathéter pour le tir au rayon laser cmportant une fibre optique contenue dans une gaine, ainsi qu'un système optique obturant l'extrémité distale de ladite gaine, de sorte que, entre l'extrémité distale de ladite fibre optique et ledit système optique, est ménagée une chambre qui est en communication avec un conduit d'amenée pour un fluide de refroidissement, et qui est pourvue d'un orifice de sortie pour ledit fluide, ledit conduit d'amenée, s'étendant parallèlement à ladite fibre optique. Ce catheter ne comporte pas de conduit de retour de sorte que le fluide est déchargé directement à l'extrémité distale du catheter.

Selon un premier mode de réalisation du cathéter conforme à l'invention, l'extrémité distale de ladite gaine est constituée par une tête tubulaire, qui est obturée par ledit système optique et dans laquelle débouchent ladite fibre optique et lesdits conduits d'amenée et de retour du fluide de refroidissement.

De préférence, lesdits conduits sont ménagés à l'intérieur de ladite gaine et relient les extrémités proximale et distale de celle-ci. Au moins l'un desdits conduits peut être coaxial à ladite fibre optique.

Dans une variante de réalisation du cathéter selon l'invention, le système optique est solidaire au moins de l'extrémité distale d'un élément de gaine intérieur contenu dans un élément de gaine extérieur, lesdits conduits étant respectivement constitués par les espaces tubulaires compris entre ladite fibre optique et ledit élément de gaine intérieur et entre lesdits éléments de gaine intérieur et extérieur, lesdits conduits communiquant entre eux grâce à un passage prévu dans l'élément de gaine intérieur au niveau de ladite chambre et ledit conduit entre lesdits éléments de gaine intérieur et extérieur étant obturé à son extrémité distale.

Les figures du dessin annexé feront bien comprendre comment l'invention peut être réalisée.

La figure 1 est une vue en coupe agrandie de l'extrémité distale d'un premier mode de réalisation du cathéter conforme à l'invention.

La figure 2 est une vue en coupe agrandie de l'extrémité distale d'une variante de réalisation.

Sur ces figures, les parties intermédiaires et les extrémités proximales des cathéters n'ont pas été représentés. Il en est de même des raccords à un générateur laser et à une source de fluide de refroidissement, disposés du côté desdites extrémités proximales.

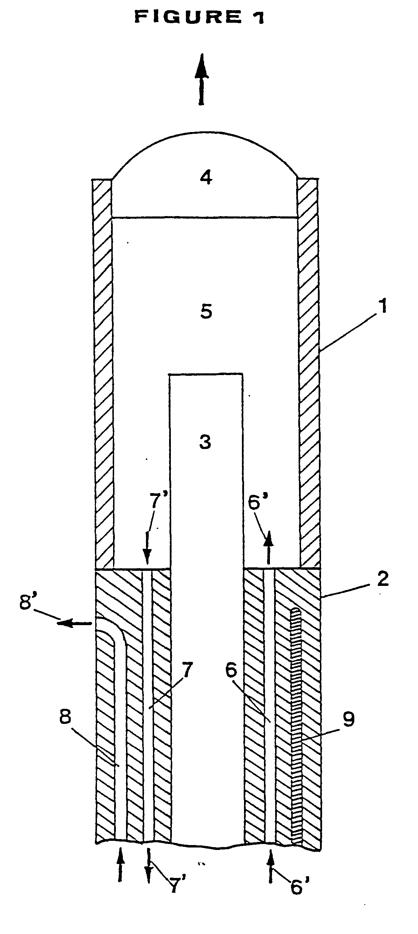

Dans le mode de réalisation de la figure 1, l'extrémité distale du cathéter est pourvue d'une tête 1, fixe ou mobile, de forme générale cylindrique en continuité avec une gaine souple 2.

Dans la tête 1 débouche une fibre optique 3 dont l'extrémité proximale (non représentée) peut être optiquement connectée à un faisceau laser, de manière connue.

La tête 1 est fermée de manière étanche par un système optique 4, destiné à protéger la fibre optique et à moduler le diamètre de l'impact des rayons.

La tête 1 est destinée à jouer le rôle de chambre 5 de transmission optique entre la fibre 3 et le système optique 4. Elle est remplie d'un fluide optiquement neutre, c'est-à-dire capable de transmettre l'énergie émise par le fibre sans être lui-même modifié ou décomposé.

Ce même fluide sert également de fluide de refroidissement de la tête 1 et de l'extrémité distale de la fibre 3. Il est donc régénéré de manière continue, par exemple par une circulation entre la tête 1 et un dispositif de pompage et de réfrigération (non représenté), circulation rendue possible grâce à un conduit d'amenée 6 et un conduit de retour 7, ménagés à l'intérieur de la gaine 2, parllèlement à la fibre 3, et débouchant dans la tête 1. Le sens de circulation du fluide peut être par exemple celui illustré par les flèches 6' et 7'.

Un fluide possédant de bonnes propriétés de transmission de la lumière et de la chaleur est par exemple l'eau distillée ou l'eau déminéralisée.

Quel qu'il soit, il est évident que le raccordement entre la tête 1 et la gaine 2 doit empêcher le sang de pénétrer dans la chambre 5 de transmission d'énergie en raison du risque d'auto-destruction du système.

Ainsi, et selon les matériaux en présence, la tête 1 peut être solidaire de la gaine 2 par soudage, collage ou friction où, si elle est prévue interchangeable, par exemple par vissage avec interposition d'un joint, par soudure à une éventuelle tige de guidage 9, ou encore par accouplement in situ dans la zone de tir.

Les matières à détruire par de la lumière doivent absorber celle-ci, sinon la destruction est impossible.

Dans le cas de plaques athéromateuses, de couleur pratiquement blanche, la lumière incidente est essentiellement réfléchie, de sorte que la réaction de destruction ne commence qu'après un temps d'amorçage assez long, une fois qu'une partie du tissu a changé de couleur. C'est notamment le cas avec des fibres optiques de petit diamètre qui ne peuvent transporter qu'une énergie faible, de l'ordre de 12 à 25 W, pour des tirs de courte durée.

Pour accélérer l'amorçage de la destruction, il peut donc être souhaitable d'injecter un colorant sur la zone à traiter. Ce colorant, venant au contact de la zone à traiter, donc injecté directement dans le vaisseau sanguin, sera par exemple de l'hémoglobine plus ou moins diluée par du sérum. Il peut être amené sur la zone à traiter par exemple, et de préférence, grâce à un troisième conduit 8, également incorporé dans la gaine 2, débouchant à proximité (en 8') de la tête 1 et alimenté en amont par un dispositif tel qu'une pompe ou une seringue (non représentée), disposé du côté proximal du cathéter.

Pour isoler la zone de tir, pour centrer le système optique et pour favoriser l'écoulement (sortant de 8') du colorant en direction de la zone à traiter, le cathéter peut être muni d'un ballonnet gonflable (non représenté), de type connu, entourant la gaine 2 en amont de la sortie 8' du colorant et alimenté en fluide de gonflement par un conduit (non représenté) également incorporé dans la gaine 2. La technique des sondes à ballonnets gonflables est bien connue, de sorte que d'autres explications sont superflues pour le spécialiste.

Ledit ballonnet, une fois gonflé, a donc pour fonction de forcer l'écoulement du colorant en direction de la zone d'impact du rayon laser, mais il sert également à bloquer le cathéter à l'intérieur du vaisseau sanguin, empêchant d'éventuels déplacements fortuits le long de son axe.

Enfin, pour remédier à la trop grande souplesse de la fibre 3 entourée de sa gaine 2, on peut encore incorporer dans ladite gaine un moyen de renfort tel que, par exemple, au moins une armature 9 constituée d'un matériau moins souple que celui de la gaine. Il peut s'agir par exemple d'une tige métallique ou d'un fil métallique enroulé en hélice, en acier inoxydable, ce qui a pour avantage supplémentaire de permettre la localisation du cathéter par radioscopie, lorsqu'il est en place dans le corps d'un patient, et peut servir pour le guidage de la sonde dans certaines artères du coeur.

Bien que, sur la figure 1, les axes longitudinaux de la fibre 3, des divers conduits 6, 7, 8 et de l'armature 9 sont tous représentés dans un même plan médian de la gaine 2, il va de soi que cette représentation a été faite ainsi pour des raisons de simplification, et qu'il est préférable de répartir les éléments 6, 7, 8 et 9, ainsi que le conduit d'alimentation d'un éventuel ballonnet (non représenté), de manière relativement régulière autour de la fibre 3 centrale, en vue de garantir une meilleure tenue de l'ensemble et d'en faciliter la fabrication.

Une autre variante encore consiste à conformer l'un des conduits 6 ou 7 non pas parallèlement et à distance de la fibre 3, mais à lui donner un diamètre supérieur à celui de la fibre 3 et à le rendre coaxial avec celle-ci: ainsi le réfrigérant est au contact de la fibre sur pratiquement toute sa longueur.

Pour assurer une position précise de l'extrémité distale de la fibre dans la tête 1, il convient alors, au voisinage de la zone de raccordement, de prévoir dans le conduit central des moyens tels que des bossages, maintenant la fibre, mais n'empêchant pas le passage du réfrigérant, ou encore de munir le conduit central d'une rainure hélicoïdale, à la manière d'un taraudage dans lequel la fibre 3 est maintenue par friction, le réfrigérant suivant une trajectoire hélicoïdale autour et au contact de la fibre.

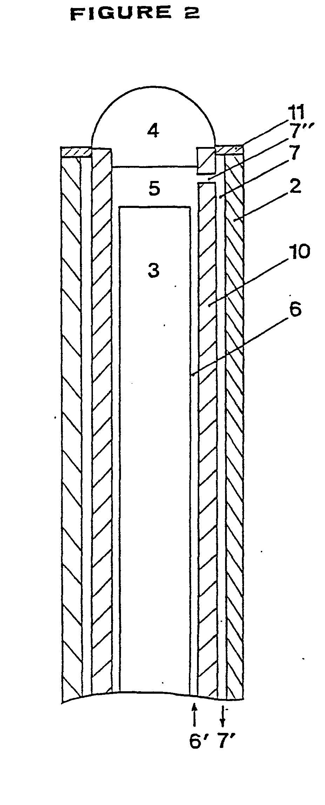

Une variante de réalisation du cathéter de l'invention est illustrée par la figure 2. Selon cette variante, le système optique 4 est solidaire d'un élément de gaine intérieur 10 contenant la fibre optique 3, ledit élément intérieur 10 étant contenu lui-même dans un élément de gaine extérieur 2. Les diamètres intérieur et extérieur de l'élément de gaine intérieur 10 sont choisis de façon telle que l'espace entre la fibre optique 3 et l'élément 10 d'une part, et l'espace entre les deux éléments 10 et 2 d'autre part, constituent des conduits coaxiaux 6 et 7 où peut circuler le liquide de refroidissement, lesdits conduits étant communi- quants grâce à un passage 7″ prévu dans la gaine 10 au niveau de la chambre optique 5. L'extrémité distale de l'élément intérieur 2 est obturée par le système optique 4.

Un dispositif d'obturation 11 assure l'étanchéité entre le circuit de refroidissement 6, 7 et l'extérieur du cathéter.

Les deux éléments de gaine 2 et 10 peuvent être éventuellement mobiles l'un par rapport à l'autre, de sorte que, dans un premier temps, seul l'élément de gaine 2 peut être amené sur le lieu de la lésion à traiter selon la technique classique du cathétérisme vasculaire, par exemple au moyen d'un guide puis, dans un deuxième temps, l'élément de gaine intérieur 10 portant le système optique 4 et contenant la fibre optique 3 est mis en place dans l'élément de gaine 2. Dans cette hypothèse, l'étanchéité étant assurée par le dispositif 11, il conviendra de préalablement purger la chambre optique par le liquide de refroidissement, afin d'en chasser toute trace de pigment sanguin avant le tir au rayon laser.

Les matériaux constitutifs des divers éléments du cathéter selon l'invention seront déterminés facilement par le spécialiste, compte tenu des diverses conditions requises: résistance mécanique, résistance thermique, inertie physiologique, inertie optique, etc.

Ainsi par exemple la tête 1 peut être en un métal, tel que l'acier inoxydable ou en matériau céramique.

Le système optique 4 peut être par exemple en saphir synthétique. La gaine ou l'élément de gaine 2 peut être en une matière plastique, telle qu'un polychlorure de vinyle, un polytétrafluoroéthylène, un polyéthylène, etc. Elle peut également être constituée d'un fil d'acier bobiné en hélice à spires jointives et être revêtue extérieurement et intérieurement de polytétrafluoroéthylène.

Le ballonnet gonflable (non représenté) peut être en polychlorure de vinyle, en silicone ou en tout matériau convenable résistant à la pression requise.

La tige 9 de renfort et de guidage peut être en acier inoxydable éventuellement revêtu de polytétrafluoroéthylène, en tungstène, etc. Elle peut ainsi être constituée d'un fil enroulé ou non autour d'une fine lame rectiligne, éventuellement mobile, de sorte que le retrait de cette dernière permet la formation d'un conduit, utilisable par exemple comme conduit de retour 7.

Un conduit supplémentaire peut éventuellement être prévu pour le lavage de la surface externe du système optique, conduit qui peut être confondu avec un conduit destiné à contenir un dispositif de guidage.

Pour des raisons évidentes de dimensions des vaisseaux à traiter, il sera en effet nécessaire, dans certains cas, de diminuer le nombre de conduits et d'utiliser, par exemple, le conduit du liquide de refroidissement 6 aussi comme conduit du fluide de gonflement du ballonnet.

Enfin, il est possible d'utiliser la gaine 2 et/ou la fibre 3 comme système d'éclairage, selon le principe bien connu des endoscopes, la fibre optique destinée au tir pouvant servir à la visualisation directe ou indirecte de la zone à traiter, grâce à un dispositif optique à double entrée.

## Revendications

1. Cathéter pour le tir au rayon laser comportant une fibre optique (3) contenue dans une gaine (2), ainsi qu'un système optique (4) obturant l'extrémité distale (1) de ladite gaine, de sorte que, entre l'extrémité distale de ladite fibre optique (3) et ledit système optique (4), est ménagée une chambre (5) qui est en communication avec un conduit d'amenée (6) pour un fluide de refroidissement, et qui est pourvue d'un orifice de sortie pour ledit fluide, ledit conduit d'amenée (6) s'étendant parallèlement à ladite fibre optique (3), caractérisé en ce que ledit orifice de sortie est relié à un conduit de retour (7) pour ledit fluide de refroidissement optiquement neutre et en ce que ledit conduit de retour (7) s'étend également parallèlement à ladite fibre optique (3).

2. Cathéter selon la revendication 1, caractérisé en ce que l'extrémité distale de ladite gaine est constituée par une tête tubulaire rigide (1) qui est obturée par ledit système optique (4) et dans laquelle débouchent ladite fibre optique (3) et lesdits conduits (6, 7) d'amenée et de retour de fluide.

3. Cathéter selon la revendication 1, caractérisé en ce que lesdits conduits (6) et (7) d'amenée et de retour du fluide de refroidissement sont ménagés à l'intérieur de la gaine (2), parallèlement et à distance de la fibre optique 3.

4. Cathéter selon la revendication 1, caractérisé en ce que au moins l'un des conduits (6, 7) d'amenée et de retour de fluide de refroidissement est coaxial avec la fibre (3) et d'un diamètre plus grand que celle-ci.

5. Cathéter selon la revendication 1, caractérisé en ce qu'il comporte un troisième conduit (8) débouchant à proximité et à l'extérieur de l'extrémité distale dudit cathéter.

6. Cathéter selon la revendication 1, caractérisé en ce que ladite gaine (1) est renforcée par au moins une armature (9) en un matériau sensiblement moins souple que le matériau de la gaine.

7. Cathéter selon la revendication 1, caractérisé en ce que le système optique est solidaire au moins de l'extrémité distale d'un élément de gaine intérieur (10) contenu dans un élément de gaine extérieur (2), lesdits conduits (6) et (7) étant respectivement constitués par les espaces tubulaires compris entre la fibre (3) et ledit élément de gaine intérieur (10) d'une part et entre lesdits éléments de gaine intérieur et extérieur (10, 2) d'autre part, lesdits conduits communiquant entre eux grâce à un passage (7″) prévu dans l'élément de gaine intérieur (10) au niveau de ladite chambre (5), et ledit conduit (7) entre lesdits éléments de gaine intérieur et extérieur étant obturé à son extrémité distale.

**Patentansprüche**

1. Katheter zur Laserstrahlbehandlung bestehend aus einer in einer Umhüllung (2) enthaltenen Lichtleitfaser (3) sowie einem optischen System (4), durch das das distale Ende (1) der Umhüllung derart abgeschlossen wird, daß zwischen dem distalen Ende der Lichtleitfaser (3) und dem optischen System (4) eine Kammer (5) ausgespart ist, die mit einer Zuführleitung (6) für ein Kühlfluid in Verbindung steht und die mit einer Ausgangsöffnung für das Fluid versehen iste, wobei die Zuführleitung (6) sich parallel zur Lichtleitfaser (3) erstreckt, dadurch gekennzeichnet, daß die Ausgangsöffnung an einer Rückführleitung (7) für das optisch neutrale Kühlfluid liegt und daß sich die Rückführleitung (7) ebenfalls parallel zur Lichtleitfaser (3) erstreckt.

2. Katheter nach Anspruch 1, dadurch gekennzeichnet, daß das distale Ende der Umhüllung gebildet ist aus einem starren rohrförmigen Kopf (1), der von dem optischen System (4) abgeschlossen ist und in den die Lichtleitfaser (3) sowie die zuführende und die rückführende Leitung des Fluids (6 bzw. 7) münden.

3. Katheter nach Anspruch 1, dadurch gekennzeichnet, daß die Zuführ- und Rückführleitungen (6 und 7) des Kühlfluids im Inneren der Umhüllung (2), parallel zu und in einem Abstand von der Lichtleitfaser (3) ausgespart sind.

4. Katheter nach Anspruch 1, dadurch gekennzeichnet, daß zumindest eine der zuführenden und rückführenden Leitungen (6, 7) des Kühlfluids mit der Lichtleitfaser (3) koaxial liegt und dieser gegenüber einen größeren Durchmesser aufweist.

5. Katheter nach Anspruch 1, dadurch gekennzeichnet, daß er eine dritte Leitung (8) aufweist, die in der Nähe und außerhalb vom distalen Ende des Katheters mündet.

6. Katheter nach Anspruch 1, dadurch gekennzeichnet, daß die Umhüllung (1) durch zumindest eine aus einem Werkstoff bestehende Armierung (9) verstärkt ist, der im wesentlichen weniger geschmeidig ist als der Werkstoff der Umhüllung.

7. Katheter nach Anspruch 1, dadurch gekennzeichnet, daß das optische System zumindest mit dem distalen Ende eines inneren Umhüllungsteiles (10) fest verbunden ist, das in einem äußeren Umhüllungsteil (2) enthalten iste, wobei die Leitungen (6) und (7) jeweils aus den röhrenförmigen Räumen, die einerseits zwischen der Lichtleitfaser (3) und dem inneren Umhüllungsteil (10) und andererseits zwischen den inneren und äußeren Umhüllungsteilen (10, 2) liegen, gebildet sind und aufgrund eines in Höhe der Kammer (5) im inneren Umhüllungsteil (10) vorgesehenen Durchgangs (7″) miteinander in Verbindung stehen und wobei die Leitung (7) zwischen dem inneren und dem äußeren Umhüllungsteil an seinem distalen Ende verschlossen ist.

**Claims**

1. Catheter for shooting a laser beam, comprising an optical fiber (3) contained in a sheath (2), as well as an optical system (4) obturating the distal end (1) of said sheath, so that, between the distal end of said optical fiber (3) and said optical fiber (4), there is formed a chamber (5) which is in communication with a supply conduit (6) for a cooling fluid, and which is provided with an outlet orifice for said fluid, said supply conduit (6) being parallel to said optical fiber (3), characterized in that said outlet orifice is connected to a return conduit (7) for said optically neutral cooling fluid and in that said return conduit (7) is also parallel to said optical fiber (3).

2. Catheter according to claim 1, characterized in that the distal end of said sheath is constituted by a rigid tubular head (1) obturated by said optical system (4) and into which open out said optical fiber (3) and said fluid supply and return conduits (6, 7).

3. Catheter according to claim 1, characterized in that said cooling fluid supply and return conduits (6, 7) are arranged inside the sheath (2), parallel to and at a distance from the optical fiber (3).

4. Catheter according to claim 1, characterized in that at least one of the cooling fluid supply and return conduits (6, 7) is coaxial with the fiber (3) and has a larger diameter than said fiber.

5. Catheter according to claim 1, characterized in that it comprises a third conduit (18) opening out in the vicinity of and outside the distal end of said catheter.

6. Catheter according to claim 1, characterized in that said sheath (1) is reinforced by at least one reinforcement (9) made of a material substantially less supple than the material of the sheath.

7. Catheter according to claim 1, characterized in that the optical system is fast with at least the distal end of an inner sheath element (10) contained in an outer sheath element (2), said conduits (6) and (7) being respectively constituted by the tubular spaces included between the fiber (3) and said inner sheath element (10), on the one hand, and between said inner and outer sheath elements (10, 2) on the other hand, said conduits communicating with each other via a passage (7″) provided in the inner sheath element (10) at the level of said chamber (5), and said conduit (7) between said inner and outer sheath elements being obturated at its distal end.

FIGURE 1

FIGURE 2